(19) Europäisches Patentamt
European Patent Office
Office européen des brevets

(11) EP 0 647 472 B1

(12) **FASCICULE DE BREVET EUROPEEN**

(45) Date de publication et mention de la délivrance du brevet: **19.07.2000 Bulletin 2000/29**

(51) Int. Cl.[7]: **B01J 25/00**, C07C 209/36

(21) Numéro de dépôt: **94401878.7**

(22) Date de dépôt: **19.08.1994**

(54) **Composition de matière utile comme catalyseur de type raney, pour l'hydrogenation d'halogenonitroaromatiques en halogenoaminoaromatiques et procédé en faisant application**

Raney Katalysator Zusammensetzung für die Hydrogenierung von Halonitroaromatischen Verbindungen und Verfahren zur Verwendung derselben

Composition of matter as Raney catalyst for hydrogenating halonitroaromatic compound and process using the same

(84) Etats contractants désignés:
**AT BE CH DE DK ES FR GB GR IE IT LI LU NL PT SE**

(30) Priorité: **07.10.1993 FR 9312149**

(43) Date de publication de la demande:
**12.04.1995 Bulletin 1995/15**

(73) Titulaire:
**CENTRE NATIONAL DE LA RECHERCHE SCIENTIFIQUE (CNRS)**
**75794 Paris Cedex 16 (FR)**

(72) Inventeurs:
- **Damon, Jean-Pierre**
  **F-38660 Le Touvet (FR)**
- **Cordier, Georges**
  **F-69340 Francheville (FR)**
- **Fouilloux, Pierre**
  **F-69300 Caluire et Cuire (FR)**
- **Marion, Philippe**
  **F-69100 Villeurbanne (FR)**

(74) Mandataire:
**Ricalens, François et al**
**RHODIA CHIMIE**
**Direction de la Propriété Industrielle**
**25, quai Paul Doumer**
**92408 Courbevoie Cédex (FR)**

(56) Documents cités:
**EP-A- 0 325 892**
**EP-A- 0 409 709**
**FR-A- 2 057 821**
**GB-A- 2 175 910**
**US-A- 4 297 509**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Xerox (UK) Business Services
2.16.7 (HRS)/3.6

## Description

Le domaine de l'invention est celui de la réduction catalytique des halogénonitroaromatiques en halogénoaminoaromatiques, par hydrogénation et à l'aide de catalyseurs à base de nickel de Raney modifié.

La présente invention concerne une composition de matière utile, de préférence, comme catalyseur de type Raney, ainsi qu'un procédé d'hydrogénation dans lequel ladite composition est mise en oeuvre.

Les catalyseurs du type nickel de Raney sont largement utilisés dans les réactions d'hydrogénation. Ils sont préparés par attaque alcaline à l'aide d'une base concentrée, d'alliages aluminium-nickel riches en aluminium. Cette attaque a pour conséquence essentielle l'élimination d'aluminium. Les catalyseurs obtenus sont constitués par des agglomérats de cristallites de nickel ayant une grande surface spécifique et un contenu résiduel en aluminium variable. Pour plus de précisions, on peut se référer, par exemple, à la littérature suivante :

- M. KHAIDAR, C. ALLIBERT, J. DRIOLE and P. GERMI - Mat. Res. Bull., 1982, 17, 329-337,
- V. BIRKENSTOCK, R. HOLM, B. REINFANDT and S. STORP - J. Catal., 1985, 93, 55-67,
- J. GROS, S. HAMAR-THIBAULT and J. C. JOUD - Surface and Interface Analysis, 1988, 11, 611-616.

La réaction de réduction des halogénonitroaromatiques, tels que le 3-chloro-4-fluoronitrobenzène, en halogénoaminoaromatiques, tels que le 3-chloro-4-fluoroaniline, est d'une grande importance en chimie organique, car elle constitue un passage clef de l'obtention de principes actifs pharmaceutiques.

L'une des pierres d'achoppement de cette réduction par hydrogénation, à l'aide de nickel de Raney ou analogues, est une réaction parasite d'hydrodéhalogénation consécutive à la réduction de la fonction nitro. Cette réaction se produit uniquement à partir de l'aniline halogénée. Cette hydrodéhalogénation du noyau aniline conduit, outre son effet négatif sur la sélectivité et le rendement de la réaction d'hydrogénation catalytique, à la formation de sous-produits particulièrement agressifs, notamment à l'encontre du catalyseur de type nickel de Raney. Ces sous-produits sont, par exemple, l'acide chlorhydrique qui a pour effet de diminuer notablement la durée de vie du catalyseur. L'hydrodéhalogénation est particulièrement critique dans le domaine des faibles concentrations en substrat.

La demande de brevet international **PCT 89/07 096** s'intéresse précisément à ce problème d'hydrogénation catalytique des composés halonitroaromatiques. Le procédé décrit dans cette demande de brevet repose sur l'utilisation d'un catalyseur de Raney à base de cobalt/aluminium/nickel/chrome. Même si ce procédé amène des améliorations par rapport à des catalyseurs du type platine/carbone activé ou nickel activé, éventuellement associés à des inhibiteurs de catalyseurs, ou bien encore par rapport à d'autres catalyseurs du type platine modifié au soufre, la réaction parasite d'hydrodéhalogénation persiste et produit encore des quantités non négligeables de composés déhalogénés et de sous-produits halogénés indésirables.

Le brevet canadien **CA 961 834** décrit des catalyseurs Ni (85 à 96 %)/Mo (0,5 à 10 %)/Al (≤ 14 %) utilisables dans l'hydrogénation catalytique, notamment de composés carbonylés comme, par exemple, l'acétone, le nitrophénolate, l'itaconate...

Il s'agit ici de substrats différents de ceux intéressant les catalyseurs et le procédé d'hydrogénation conformes à l'invention. Ces catalyseurs ne sont, ni sélectifs, ni significativement actifs vis-à-vis de radicaux nitro portés par des groupements aromatiques. En outre, ce brevet n'enseigne, et pour cause, rien en ce qui concerne l'hydrodéhalogénation.

Dans le même ordre d'idées, l'article de S. **HAMAR-THIBAULT** et al., J. Chim. Phys. (1991) 88, 219-232, concerne des catalyseurs de Raney dopés au molybdène et appliqués dans l'hydrogénation de l'acétophénone en phase liquide. Ici encore, on ne se trouve pas confronté au problème de l'hydrodéhalogénation. En outre, ce document enseigne que plus la teneur en molybdène du catalyseur augmente, plus l'activité intrinsèque et la spécificité vis-à-vis des sites à hydrogéner, i. e. les carbonyles, diminuent.

Parmi les autres produits de départ d'hydrogénation catalytique qui ne sont pas concernés par la réaction parasite d'hydrodéhalogénation, on peut citer les dinitroaromatiques réduits en diaminoaromatiques du procédé décrit dans le brevet **DE 35 37 247**. Ce procédé d'hydrogénation fait intervenir les catalyseurs à base de nickel de Raney dopés au molybdène. Ces catalyseurs finis sont pauvres en aluminium, relativement à la quantité de Mo présente, comme cela est illustré par le rapport Al/Mo, qui est inférieur à 1 (Al et Mo étant exprimés % en poids) pour les catalyseurs de Raney décrits dans ce document.

Enfin, on connaît, également, des catalyseurs de Raney dopés par un élément de transition métallique. Ces catalyseurs d'hydrogénation monodopés NiAl/métal sont utilisés dans la réduction sélective du parachloronitrobenzène en parachloroaniline. La présence d'un dopant métallique dans le catalyseur de Raney entraîne une baisse de sélectivité et une baisse d'activité catalytique. De plus, le dopant semble avoir pour incidence une augmentation de l'hydrodéhalogénation. Cette dernière reste même notable pour des concentrations fortes en parachloronitrobenzène (cas du fer

et du chrome). Ainsi, dans un environnement halogénoaromatique, le dopage métallique des catalyseurs de Raney ne paraît pas souhaitable.

Dans cet état de la technique, l'un des objectifs essentiels de la présente invention est de fournir une composition de matière utile, notamment comme catalyseur de type Raney, pour l'hydrogénation catalytique d'halogénonitroaromatiques en halogénoaminoaromatiques, qui soit très active et sélective et qui n'entraîne pas de réactions parasites secondaires d'hydrodéhalogénation ou, pour le moins, qui les limites au maximum.

Un autre objectif de l'invention est de fournir une composition de matière utile comme catalyseur de Raney qui bénéficie d'une excellente tenue chimique, aussi bien lors de son stockage que lors de la réaction. En effet, le catalyseur doit, de préférence, être insensible aux réactifs et aux produits de l'hydrogénation catalytique.

Un autre objectif de l'invention est de fournir une composition de matière utile comme catalyseur de Raney qui puisse être fabriquée, de manière aisée et économique.

C'est ainsi qu'après de nombreux études et essais, la Demanderesse a eu le mérite de mettre en évidence qu'il convenait de retenir le molybdène à titre de dopant dans un catalyseur de Raney classique et que ce molybdène devait être, avantageusement, présent dans le catalyseur en quantité précise et donnée, par rapport au nickel et à l'aluminium.

Il s'ensuit que les susdits objectifs, parmi d'autres, sont atteints par la présente invention qui concerne une composition de matière utile comme catalyseur de type Raney pour l'hydrogénation catalytique d'halogénonitroaromatiques en halogénoaminoaromatiques ayant pour caractéristiques de pouvoir être issue d'un alliage essentiellement constitué par Ni/Al/Mo et d'être défini par un rapport pondéral Al/Mo supérieur ou égal à 1 de préférence à 2 et, plus préférentiellement encore est compris entre 2,5 et 3,5.

Un tel catalyseur de Raney dopé molybdène s'avère être très actif et sélectif. De plus, il n'induit qu'une très faible hydrodéhalogénation. Ce phénomène parasite est, en effet, au moins deux fois moins important sur le plan quantitatif que dans l'art antérieur.

En outre, on observe que ce catalyseur n'est pratiquement pas attaqué au cours de la réaction d'hydrogénation catalytique. Il bénéficie donc d'une très bonne stabilité chimique dans le temps.

Il est hautement préférable que le rapport massique [expression équivalente à l'expression "rapport pondéral" considérée comme fautive par les puristes (En effet, il s'agit de définir un rapport de quantité de matière et le rapport des poids est un rapport de forces)] Mo/(Ni Al) soit au moins égal à 5 %.

Il est également souhaitable que le rapport massique Mo/Ni soit au moins égal à 3/40, de préférences à 0,1.

Le rapport Ni/(Ni+Al+Mo) est avantageusement au moins égal à 0,5. avantageusement une caractéristique importante est que la composition selon l'invention peut - être réalisée par attaque alcaline l'un alliage mère contenant plus d' aluminium que ladite composition.

Lorsque l'on ne considère que les trois éléments clefs nickel, molybdène et Aluminium, la composition catalytique selon l'invention est caractérisée par le fait qu'elle contient Ni/Al/Mo dans les proportions pondérales - exprimées en % poids - suivantes :

- $50 \le Ni \le 78$, de préférence $60 \le Ni \le 70$,
- $20 \le Al \le 40$, de préférence $25 \le Al \le 35$,
- et $2 \le Mo \le 20$, de préférence $5 \le Mo \le 15$.

Les impuretés sont celles usuelles en la matière( en nature et en proportion).

Une composition catalytique plus particulièrement préférée pour le catalyseur de Raney dopé molybdène sont, celles qui correspondent à une composition d'alliage précurseur Ni/Al/Mo, respectivement de 44, 38 et 18 % en poids.

Il apparaît qu'il reste, après attaque alcaline, environ la moitié de l'aluminium, tandis que les deux tiers environ du molybdène est lessivée par la soude. on obtient des compositions selon la présente invention ayant en masse, après arrondi mathématique, trois fois autant d'aluminium que de molybdène et sept fois autant de nickel que de molybdène (rapport 7/3/1). en particulier la composition en proportions massiques Ni/Al/Mo = 63/28/9 utilisée dans l'exemple 1.

Les proportions relatives des constituants de la composition selon l'invention, et de l'alliage duquel elle peut être issue, sont données aux impuretés près. En d'autres termes, cela signifie qu'il convient de tenir compte d'une certaine tolérance aux impuretés (notamment métalliques) dans la composition et dans l'alliage.

La composition catalytique selon l'invention (dénommée également et indifféremment "catalyseur" dans le présent exposé) peut être utilisée pure ou en association avec d'autres matériaux, tels que des supports inertes, dans les conditions d'usage. Un tel catalyseur peut se présenter sous différentes formes.

Il peut s'agir, par exemple, d'un substrat monolithique (nid d'abeilles ou autres) en composition Ni/Al/Mo, ou d'un substrat monolithique revêtu d'une couche en composition Ni/Al/Mo, ou bien encore se présenter sous la forme de produits divisés en, ou revêtus de, composition Ni/Al/Mo.

Par forme divisée, on entend des produits pulvérulents (poudres) et également les articles obtenus par mise en forme de ces produits (billes, pastilles, boulettes, granulés, extrudés, agglomérés et autres, de section circulaire, ovale, trilobée ou multilobée, pleine ou creuse).

Les catalyseurs de type billes, pastilles et autres, présentent l'avantage de pouvoir être séparés ultérieurement du milieu de réaction très rapidement, par simple décantation. Les catalyseurs de type pulvérulent nécessitent généralement, pour leur séparation, une étape de filtration.

Bien entendu, tous les catalyseurs précités sont choisis avec une surface spécifique convenant pour l'application considérée. Dans la pratique, on peut mettre en oeuvre un composition Ni/Al/Mo dont la surface spécifique, mesurée selon la méthode BET (BRUNAUER, EMMETT et TELLER) peut varier d'un dixième à plusieurs centaines, voire quelques milliers de mètres carrés par gramme et, en général, de 1 à 500 $m^2/g$. Elle est, le plus souvent et de préférence, comprise entre 10 et 100 $m^2/g$.

A cet effet, on pourra utiliser, soit des carbures de tungstène disponibles dans le commerce, soit des carbures de tungstène que l'on aura synthétisés selon tout procédé connu en soi.

L'avantageuse sélectivité du catalyseur selon l'invention s'exprime notamment lors des réactions d'hydrogénation. ainsi selon la présente invention l'on a trouvé un réactif sélectif d'hydrogénation comportant une composition catalytique et de l'hydrogène dont la pression partielle est comprise entre 1 et 100, de préférence entre 5 et 50 et, plus préférentiellement encore entre 15 et 25 bar (1 bar = $10^5$ Pa).

Cette sélectivité est particulièrement nette vis-à-vis de produits de départ choisis parmi les dérivés halogéno-nitro(ou nitroso)-aromatiques.ces composés présente avantageusement au moins un groupe nitro (ou nitroso) directement lié à un chaînon carboné aromatique et au moins un halogène également directement lié à un chaînon carboné aromatique. De préférence au moins un halogène et au moins un nitro sont liés au même radical aromatique (aryle), lequel peut être à un noyau ou à plusieurs noyaux condensés.

Avantageusement Il y a au plus 3, de préférence au plus 2, groupes nitro ou nitroso, par radical aromatique.

Avantageusement Il y a au plus 4, de préférence au plus 3, atomes d'halogène par radical aromatique.

Ainsi le pouvoir catalytique des compositions selon la présente invention est particulièrement intéressant pour les dérivés halogéno-nitro(ou nitroso)aromatiques possédant un ou plusieurs radicals(aux) aromatique(s), de préférence un seul et, plus préférentiellement encore, répondant à la formule suivante :

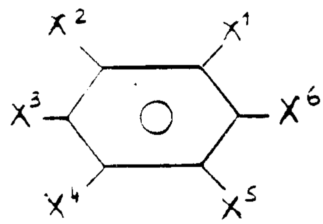

- dans laquelle $X^1$ à $X^6$, identiques ou différents, représentent H, un halogène, de préférence F, Cl, Br, un radical $NO_x$, avec x = 1 ou 2, OR, avec R = H , alcoyle, linéaire ou branché, alcényle, aryle ou aralcoyle, ou un métal alcalin, de préférence un radical méthyle, éthyle, propyle, butyle, phényle, benzyle, ou le Naphtyle,
- avec au moins l'un des radicaux $X^1$ à $X^6$ = halogène et au moins un autre = $NO_x$, avec x = 1 ou 2.

Parmi les halogénonitroaromatiques donnant les meilleurs résultats, on peut citer : le 3-chloro-4-fluoronitrobenzène, le 3,4 et le 3,5-dichloronitrobenzène, le 2,4-difluoronitrobenzène et le 4-fluoronitrobenzène.

Parmi les substrats possibles, il convient de citer les mélanges contenant un ou plusieurs dérivés ci dessus. En effet les réactions conduisant au substrats donnent parfois des mélanges difficiles à séparer mais qui une fois hydrogénés sont plus aisés à séparer.

La présente invention concerne, également, un procédé d'hydrogénation catalytique dans lequel la composition catalytique décrite ci-dessus est utilisée. Il va de soi que ce procédé peut être mis en oeuvre selon un mode de fonctionnement continu ou discontinu.

Les quantités de catalyseur mises en oeuvre dans ce nouveau procédé peuvent varier en fonction de multiples facteurs comme, par exemple, l'activité spécifique du catalyseur considéré, les conditions réactionnelles, telles que la température, la pression, le temps de réaction, la nature des halogénonitroaromatiques à hydrogéner et la méthodologie retenue.

Avantageusement, lorsqu'on opère en mode discontinu, le catalyseur est présent à raison de 10 % en poids

au plus par rapport à l'halogénonitroaromatique de départ, de préférence à raison de 2 à 5 % en poids.

Pour assurer une bonne sélectivité du catalyseur, en mode discontinu, il est avantageux de prévoir, dans le milieu réactionnel, une quantité d'halogénonitroaromatiques de départ qui reste supérieure ou égale à 5 ppm, de préférence à 10 ppm et, plus préférentiellement encore, supérieure à 100 ppm.

En mode discontinu, ces seuils quantitatifs peuvent servir de référence pour délimiter la durée réactionnelle.

L'hydrogénation s'effectue, en phase liquide, dans des conditions de température et de pression que l'homme du métier peut aisément faire varier dans une large mesure.

Par exemple, l'hydrogénation peut être effectuée :

- sous une pression en $H_2$, $P_{H2}$ comprise entre 1 et 100, de préférence entre 5 et 50 et, plus préférentiellement encore entre 15 et 25 bar (1 bar = $10^5$ Pa),
- à une température réactionnelle comprise entre 25 et 150, de préférence entre 40 et 120, plus préférentiellement encore entre 55 et 80° C.

En ce qui concerne le milieu réactionnel, on préfère, conformément à l'invention, employer un solvant inerte pour la solubilisation de l'halogénonitroaromatique de départ.

Dans le choix du milieu solvant réactionnel, il est également préférable de tenir compte de la solubilité de l'halogénoaniline visée.

En effet, étant donné que, grâce à l'invention, l'hydrodéhalogénation est limitée, la production d'halogénoaniline s'en trouve augmentée et il est intéressant que toute cette production reste rassemblée dans le solvant, de façon à en faciliter la récupération.

Avantageusement, ce milieu solvant réactionnel est constitué par au moins un alcool primaire ou secondaire ayant jusqu'à environ 4 atomes de carbone et, plus particulièrement, par le méthanol, l'éthanol, l'isopropanol. Des solvants aprotique polaires organiques, tels que le diméthylacétamide et le tétrahydrofurane, peuvent être utilisés en combinaison avec l'alcool quand les caractéristiques de solubilité des réactifs l'exigent.

Il est à noter que ce milieu réactionnel contient nécessairement de l'eau qui est un produits inévi de la réaction considérée. Il est, par ailleurs, possible de prévoir la mise en oeuvre de quantité variables d'eau, à titre de cosolvant. En pratique, le milieu solvant réactionnel est en général un alcool de 1 à 10, avantageusement de 1 à 4 atomes de carbone, de préférence le méthanol ou l'éthanol.

Les dispositifs pour la mise en oeuvre du procédé, susceptibles d'être utilisés, sont conventionnels.

Il est important de souligner que le catalyseur et le procédé selon l'invention procurent d'excellentes sélectivités d'hydrogénation des halogénonitroaromatiques en halogénoaminoaromatiques, tout en minimisant les réactions indésirables d'hydrodéhalogénation. Ces dernières sont connues pour perturber la réaction d'hydrogénation. En mode discontinu, cette perturbation intervient à la fin de la réaction, i. e. au moment où les concentrations en produits de départ deviennent faibles. En mode continu, l'effet néfaste de l'hydrodéhalogénation (qui produit un acide Halohydrique susceptible d'attaquer certains éléments du catalyseur) se fait sentir lorsque les concentrations en dérivés nitrés sont inférieures ou égales à 500 ppm et souvent à 200 ppm.

L'une des applications particulièrement intéressante de l'invention est la production d'halogénoaminoaromatiques utiles, notamment, dans le domaine pharmaceutique et agrochimique.

L'invention sera mieux comprise et d'autres de ses avantages et variantes de mise en oeuvre ressortiront bien des exemples qui suivent.

**EXEMPLES**

**EXEMPLE 1: PREPARATION DES CATALYSEURS.**

Le nickel de Raney de référence est préparé par attaque alcaline d'un alliage commercial Ni-Al (50-50 % en poids). Cet alliage précurseur est constitué d'un mélange de phases $NiAl_3$, $Ni_2Al_3$, NiAl et de l'eutectique, qui ont des réactivités différentes vis-à-vis de la soude.

Les catalyseurs mis en oeuvre dans ces exemples sont issus d'alliages précurseurs cristallisés relativement riches en nickel proche de la base $Ni_2Al_3$, dopés par l'élément d'addition molybdène : $Ni_{2-x}/Al_3/Mo_x$, avec $x = 0,4 \pm 0,05$.

Le protocole d'attaque alcaline est le suivant : 60 g de précurseur sont introduits par petites quantités pendant une durée d'environ 40 min. dans un ballon à trois cols, surmonté d'un réfrigérant, contenant 400 ml d'une solution de soude 6N bouillante. La solution est maintenue à reflux deux heures. Ensuite, le solide est lavé par une solution de soude N bouillante et de nouveau porté 2 h à ébullition dans une solution NaOH 6N. Le catalyseur est ensuite lavé dans plusieurs solutions NaOH de concentrations décroissantes (3N, 2N et N). Il est stocké dans une solution de soude N.

**EXEMPLE 2: HYDROGENATION CATALYTIQUE 3-4 CFNB.**

**A - APPAREILLAGE:**

L'appareil utilisé dans l'hydrogénation du 3-4 CFNB en phase liquide se compose :

- d'un réacteur en acier inox type SOTELEM, de contenance 250 ml, muni d'un chauffage à circulation d'eau et d'un système d'agitation (turbine à pales entraînée par un système magnétique) ; il est équipé d'un septum pour l'introduction des réactifs, d'un manomètre pour fixer la pression de gaz dans le réacteur et d'un système de vannes pour l'introduction de l'hydrogène, pour les purges ($V_2$) et pour les prélèvements ($V_3$),
- d'un régulateur de pression,
- d'une réserve d'hydrogène équipée d'un manomètre et d'une vanne pour isoler le système.

**B - MODE OPERATOIRE:**

**(i)** MISE EN OEUVRE DU CATALYSEUR:

la réaction. 4 g de catalyseur sont pesés sous méthanol, puis introduits dans le réacteur avec 130 ml du méthanol. L'ensemble est purgé par un courant d'hydrogène ($P_{H2}$ = 1 bar) de 150 ml x min.$^{-1}$ pendant 10 min.

**(i.i)** MISE EN OEUVRE DE LA REACTION D'HYDROGENATION:

La température du milieu réactionnel est portée à 60° C et la pression totale à 18 bars avec $H_2$.
La vitesse d'agitation est maintenue à 650 tr x min.$^{-1}$.
Ces grandeurs resteront constantes pendant toute la durée de l'essai (5 h).
On injecte le réactif (3-4 CFNB), dissous en mélange 50/50 dans le méthanol, dans le réacteur à travers le septum, à l'aide d'une pompe, au débit de 40 g/h.
L'instant de mise en route de l'agitation est le temps zéro de la réaction. Les conditions expérimentales les plus fréquemment utilisées sont :

- masse de catalyseur : 4 g,
- volume total = 150 ml,
- 3-4 CFNB/solvant = 40 g/h,
- $P_{H2}$ = 17,5 bar,
- vitesse d'agitation = 650 tr x min.$^{-1}$,

**C - ANALYSE DU MILIEU REACTIONNEL:**

L'évolution de la réaction est suivie en analysant, par chromatographie en phase gazeuse, des échantillons du mélange réactionnel prélevés à intervalles réguliers au cours de la réaction :

- chromatographe à ionisation de flamme Perkin-Elmer 8410,
- température de l'injecteur : 220° C,
- température du détecteur : 230° C,
- colonne utilisée : alcomine 20 %, Chromosorb GNAW 80/100, d'une longueur de 4 m et d'un diamètre de 1/8e de pouce,
- température de la colonne : 80° C,
- débit du gaz vecteur ($N_2$) : 20 ml x min.$^{-1}$,
- volume injecté : 1 µl.

La teneur en dérivés nitrés non transformés est suivie par polarographie. La sensibilité de cette méthode de mesure est supérieure ou égale à 5 ppm.

**D - COMPOSES MIS EN OEUVRE:**

Le catalyseur est le Ni-Raney dopé Mo, préparé selon la méthodologie décrite dans l'exemple 1 et de composition suivante : Ni/Al/Mo = 63/28/9 en poids.
Le substrat de départ est le 3-4-chlorofluoronitrobenzène titrant à 98,5 %.
Le milieu solvant réactionnel est constitué par du méthanol PROLABO, qualité RP.

**E - RESULTATS:**

Le tableau 1 ci-dessous donne les résultats obtenus.
L'hydrodéchloration est reflétée par la quantité des chlorures produits ramenée à un pourcentage molaire par rapport aux dérivés nitrés engagés dans l'hydrogénation. Il n'y a pas d'autres produits organiques formés que l'halogénoaniline recherchée, les autres halogénoanilines analogues et que l'aniline.

TABLEAU 1

| TEMPS (h) | Poids de nitré hydrogéné (g) | Teneur en nitré (ppm) | % amine sortie hydrogénation | % HDC hydrodéchloration |
|---|---|---|---|---|
| 0 | 0 | 0 | 0 | 0 |
| 1 | 24 | 0 | 4,8 | 3,4 |
| 2 | 44 | 5 | 24,5 | 0,8 |
| 3 | 64 | 55 | 32,7 | 0,8 |
| 4 | 85 | 230 | 37,2 | 0,6 |
| 5 | 106 | 255 | 39,2 | 0,5 |

Il a été procédé, par ailleurs, à des analyses sur les bruts d'hydrogénation et il n a été retrouvé qu'environ 9 ppm de nickel et moins de 1 ppm de molybdène ainsi que d'aluminium. Il faut donc en conclure que le catalyseur n'est pas attaqué lors de la réaction. Cela illustre un autre avantage conséquent du catalyseur suivant l'invention.

**EXEMPLE 3.**

On opère comme il est dit dans l'exemple 2, à la différence que l'on remplace le 3-chloro, 4-fluoronitrobenzène par le 3,4-dichloronitrobenzène.
Après 11 h d'hydrogénation en continu à 75° C et 15 bars de pression totale, le rendement de l'hydrodéchloration est stable à 0,9 % et la concentration de l'halogénonitrobenzène résiduaire est ≤ 100 ppm.

**EXEMPLE 4.**

On opère comme il est dit dans l'exemple 3, à la différence que l'halogénonitrobenzène hydrogéné est le 3,5 dichloronitrobenzène.
Après le même temps d'hydrogénation, dans les mêmes conditions et pour la même teneur en composé nitré résiduaire, le rendement de l'hydrodéchloration est de 0,3 %.

**EXEMPLE 5.**

On opère comme il est dit dans l'exemple 2, à la différence que l'halogénonitrobenzène à hydrogéner est le 4-fluoronitrobenzène.
L'hydrogénation est conduite à 100 ° C sous 20 bars de pression totale.
La quantité de catalyseur, la nature et la quantité du solvant mises en oeuvre sont inchangées.
La vitesse d'injection du mélange 4 fluoronitrobenzène/méthanol (50/50 p/p) est portée à 100 g x $h^{-1}$.
La teneur en 4-fluoronitrobenzène non transformé est inférieure à 100 ppm et aucune hydrodéfluoration n'est détectée.

**EXEMPLE 6.**

On opère comme dans l'exemple 5, à la différence qu'on remplace le 4-fluoronitrobenzène par le 2,4-difluoronitrobenzène.

Les résultats sont identiques à ceux de l'exemple 5, aucune hydrofluoration n'est détectée.

**EXEMPLE 7: CONTRE-EXEMPLE.**

On opère comme il est dit dans l'exemple 2, mais on remplace le catalyseur de l'invention par un catalyseur nickel de Raney classique ne contenant pas d'autre métal que l'aluminium résiduaire (7,8 % p/p) le rendement de l'hydrodéchloration est de 6 % après 5 h de réaction.

Le taux de dérivé nitré résiduaire est voisin de 200 ppm.

**EXEMPLE 8: CONTRE-EXEMPLE.**

On répète l'exemple 7, mais le catalyseur est cette fois un nickel de Raney contenant 0,8 % de fer, 2 % de chrome et 8,2 % d'aluminium.

Le rendement de l'hydrodéchloration atteint cette fois 7,5 %, pour une teneur résiduaire en composé nitré voisine de 150 ppm.

**Revendications**

1. Procédé d'hydrogénation catalytique d'un substrat halogénonitroaromatique en halogénoaminoaromatique, mis en oeuvre à l'aide d'une composition de matière utile comme catalyseur de type Raney caractérisée par le fait qu'elle est susceptible d'être obtenue par attaque alcaline d'un alliage essentiellement constitué par Ni/Al/Mo et par le fait que le rapport pondéral Al/Mo de cette composition est supérieur ou égal à 1, de préférence à 2 et, plus préférentiellement encore, est compris entre 2 et 3.

2. Procédé selon la revendication 1, caractérisé en ce que l'on effectue l'hydrogénation sous une pression en $H_2$, $P_{H2}$ comprise entre 1 et 100, de préférence entre 5 et 50 et, plus préférentiellement encore, entre 15 et 25 bar, et à une température comprise entre 25 et 150, de préférence entre 40 et 120 et, plus préférentiellement encore, entre 55 et 80°C.

3. Procédé selon les revendications 1 et 2, caractérisé en ce que le milieu solvant réactionnel est de nature alcoolique, de préférence méthanolique ou éthanolique.

4. Procédé selon les revendications 1 à 3, caractérisé en ce que l'on mène la réaction de manière que la quantité de substrat halogénonitroaromatiques reste supérieure ou égale à celle qui correspond à une concentration de 5 ppm, de préférence à 10 ppm et, plus préférentiellement encore supérieure ou égale à 100 ppm.

5. Procédé selon les revendications 1 à 4, caractérisé en ce que le substrat halogénonitroaromatiques possèdent un ou plusieurs radicals(aux) aromatique(s), de préférence un seul.

6. Procédé selon les revendications 1 à 5, caractérisé en ce que le substrat halogénonitroaromatique répond à la formule suivante :

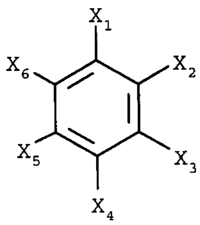

- dans laquelle $X^1$ à $X^6$ sont identiques ou différents et représentent H, un halogène, de préférence F, Cl, Br, un

radical $NO_x$, avec x = 1 ou 2, OR, avec R = H , alcoyle, linéaire ou branché, alcényle, aryle ou aralcoyle, ou bien encore métal alcalin, de préférence un radical méthyle, éthyle, propyle, butyle, phényle, benzyle, ou le naphtyle,

- avec au moins l'un des radicaux $X^1$ à $X^6$ = halogène et au moins un autre = $NO_x$ (x = 1 ou 2).

**7.** Procédé selon les revendications 1 à 6, caractérisé en ce que le substrat halogénonitroaromatique est :

- le 3-chloro-4 fluoronitrobenzène,
- le 3,4-dichloronitrobenzène,
- le 3,5-dichîoronitrobenzène,
- le 2,4-difluoronitrobenzène,
- et le 4-fluoronitrobenzène.

**8.** Composition de matière utile comme catalyseur de type Raney, pour l'hydrogénation d'halogénonitroaromatiques en halogénoaminoaromatiques, caractérisée par le fait qu'elle est susceptible d'être obtenue par attaque alcaline d'un alliage essentiellement constitué par Ni/Al/Mo, par le fait que le rapport pondéral Al/Mo de cette composition est supérieur ou égal à 1 et par le fait que le rapport massique Mo/(Ni + Al) soit au moins égal à 5 %.

**9.** Composition selon la revendication 8, caractérisée par le fait que le rapport pondéral Al/Mo de cette composition est supérieur ou égal à 2 et de préférence est compris entre 2 et 3.

**10.** Composition selon les revendications 8 et 9, caractérisée par le fait que le rapport massique Mo/Ni est au moins égal à 3/40, de préférence à 0,1.

**11.** Composition selon les revendications 8 à 10, caractérisée par le fait que le rapport Ni/(Ni+Al+Mo) est avantageusement au moins égal à 0,5.

**12.** Composition selon les revendications 8 à 11, caractérisée par le fait qu'elle contient Ni/Al/Mo dans les proportions pondérales - exprimées en % poids - suivantes :

- $50 \leq Ni \leq 78$, de préférence $60 \leq Ni \leq 70$;
- $20 \leq Al \leq 40$, de préférence $25 \leq Al \leq 35$,
- et $5 \leq Mo \leq 20$, de préférence $5 \leq Mo \leq 15$.

**13.** Réactif d'hydrogénation comportant une composition catalytique selon les revendications 8 à 12 et de l'hydrogène dont la pression partielle est comprise entre 1 et 100, de préférence entre 5 et 50 et, plus préférentiellement encore entre 15 et 25 bar (1 bar= $10^5$Pa).

## Claims

**1.** Process for the catalytic hydrogenation of a lonitroaromatic substrate to a haloaminoaromatic substrate carried out with the help of a composition of matter of use as catalyst of Raney type, characterized in that the composition is capable of being obtained by alkaline attack on an alloy composed essentially of Ni/Al/Mo and in that the Al/Mo ratio by weight of this composition is greater than or equal to 1, preferably greater than or equal to 2 and more preferably still is between 2 and 3.

**2.** Process according to Claim 1, characterized in that the hydrogenation is carried out under an $H_2$ pressure, $P_{H2}$, of between 1 and 100, preferably between 5 and 50 and more preferably still between 15 and 25 bar and at a temperature of between 25 and 150, preferably between 40 and 120 and more preferably still between 55 and 80°C.

**3.** Process according to Claims 1 and 2, characterized in that the reaction solvent medium is alcoholic in nature, preferably methanolic or ethanolic in nature.

**4.** Process according to Claims 1 to 3, characterized in that the reaction is carried out so that the amount of halonitroaromatic substrate remains greater than or equal to that which corresponds to a concentration of 5 ppm, preferably to 10 ppm and more preferably still greater than or equal to 100 ppm.

**5.** Process according to Claims 1 to 4, characterized in that the halonitroaromatic substrate possesses one or more aromatic radical(s), preferably a single aromatic radical.

**6.** Process according to Claims 1 to 5, characterized in that the halonitroaromatic substrate corresponds to the following formula:

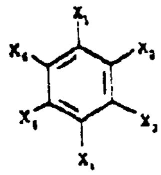

- in which $X^1$ to $X^6$ are identical or different and are H, a halogen, preferably F, Cl or Br, an $NO_x$ radical with x = 1 or 2, or OR with R = H , alkyl, which may be linear or branched, alkenyl, aryl, aralkyl or alkali metal, preferably a methyl, ethyl, propyl, butyl, phenyl, benzyl or naphthyl radical,
- with at least one of the $X^1$ to $X^6$ radicals being halogen and at least one other being $NO_x$ (x = 1 or 2).

**7.** Process according to Claims 1 to 6, characterized in that the halonitroaromatic substrate is:

- 3-chloro-4-fluoronitrobenzene
- 3,4-dichloronitrobenzene,
- 3,5-dichloronitrobenzene,
- 2,4-difluoronitrobenzene,
- and 4-fluoronitrobenzene.

**8.** Composition of matter or use as catalyst of Raney type for the hydrogenation of halonitroaromatics to haloaminoaromatics, characterized in that it is capable of being obtained by alkaline attack on an alloy composed essentially of Ni/Al/Mo, in that the Al/Mo ratio by weight of this composition is greater than or equal to 1 and in coat the Mo/(Ni + Al) ratio by mass is at least equal to 5%.

**9.** Composition according to Claim 8, characterized in that the Al/Mo ratio by weight of this composition is greater than or equal to 2 and is preferably between 2 and 3.

**10.** Composition according to Claims 8 and 9, characterized in that the Mo/Ni ratio by mass is at least equal to 3/40, preferably to 0.1.

**11.** Composition according to Claims 8 to 10, characterized in that the Ni/(Ni + Al + Mo) ratio is advantageously at least equal to 0.5.

**12.** Composition according to Claims 8 to 11, characterized in that it comprises Ni/Al/Mo in the following proportions by weight, expressed as weight%:

- 50 = Ni = 78, preferably 60 = Ni = 70;
- 20 = Al = 40, preferably 25 = Al = 35,
- and 5 = Mo = 20, preferably 5 = Mo = 15.

**13.** Hydrogenation reactant comprising a catalytic composition according to Claims 8 to 12 and hydrogen, the partial pressure of which is between 1 and 100, preferably between 5 and 50 and more preferably still between 15 and 25 bar (1 bar = $10^5$ Pa).

**Patentansprüche**

**1.** Verfahren zur katalytischen Hydrierung eines halogennitroaromatischen Substrats zu einem Halogenaminoaromaten mit einer zur Verwendung als Raney-Katalysator geeigneten Stoffzusammensetzung, dadurch gekennzeichnet, daß diese Zusammensetzung durch alkalischen Angriff auf eine im wesentlichen aus Ni/Al/Mo bestehende Legierung erhältlich ist und ein Al/Mo-Gewichtsverhältnis größer gleich 1, vorzugsweise größer gleich 2 und besonders bevorzugt zwischen 2 und 3 aufweist.

**2.** Verfahren nach Anspruch 1, dadurch gekennzeichnet, daß man die Hydrierung bei einem $H_2$-Druck $P_{H2}$ zwischen 1 und 100 bar, vorzugsweise zwischen 5 und 50 bar und besonders bevorzugt zwischen 15 und 25 bar, und einer Temperatur zwischen 25 und 150°C, vorzugsweise zwischen 40 und 120°C und besonders bevorzugt zwischen 55 und 80°C durchführt.

**3.** Verfahren nach den Ansprüchen 1 und 2, dadurch gekennzeichnet, daß man als Reaktionslösungsmittel einen Alkohol, vorzugsweise Methanol oder Ethanol, einsetzt.

**4.** Verfahren nach den Ansprüchen 1 bis 3, dadurch gekennzeichnet, daß man die Reaktion so führt, daß die Menge an halogennitroaromatischem Substrat größer oder gleich der Menge, die einer Konzentration von 5 ppm entspricht, vorzugsweise größer gleich 10 ppm und besonders bevorzugt größer gleich 100 ppm bleibt.

**5.** Verfahren nach den Ansprüchen 1 bis 4, dadurch gekennzeichnet, daß man ein halogennitroaromatisches Substrat verwendet, das einen oder mehrere aromatische Reste, vorzugsweise nur einen aromatischen Rest, aufweist.

**6.** Verfahren nach den Ansprüchen 1 bis 5, dadurch gekennzeichnet, daß man ein halogennitroaromatisches Substrat der folgenden Formel einsetzt:

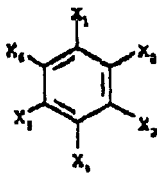

- worin $X^1$ bis $X^6$ gleich oder verschieden sind und für H, Halogen, vorzugsweise F, Cl, Br, einen Rest $NO_x$ mit x = 1 oder 2, OR mit R = H , lineares oder verzweigtes Alkyl, Alkenyl, Aryl oder Aralkyl oder auch ein Alkalimetall, vorzugsweise einen Methyl-, Ethyl-, Propyl-, Butyl-, Phenyl-, Benzyl- oder Naphthylrest stehen,
- wobei mindestens einer der Reste $X^1$ bis $X^6$ für Halogen steht und mindestens ein anderer Rest für $NO_x$ (x = 1 oder 2) steht.

**7.** Verfahren nach den Ansprüchen 1 bis 6, dadurch gekennzeichnet, daß man als halogennitroaromatisches Substrat einsetzt:

- 3-Chlor-4-fluornitrobenzol,
- 3,4-Dichlornitrobenzol,
- 3,5-Dichlornitrobenzol,
- 2,4-Difluornitrobenzol und
- 4-Fluornitrobenzol.

**8.** Zur Verwendung als Raney-Katalysator geeignete Stoffzusammensetzung zur Hydrierung von Halogennitroaromaten zu Halogenaminoaromaten, dadurch gekennzeichnet, daß sie durch alkalischen Angriff auf eine im wesentlichen aus Ni/Al/Mo bestehende Legierung erhältlich ist, ein Al/Mo-Gewichtsverhältnis größer gleich 1 und ein Mo/(Ni+Al)-Massenverhältnis von mindestens 5% aufweist.

**9.** Zusammensetzung nach Anspruch 8, dadurch gekennzeichnet, daß deren Al/Mo-Gewichtsverhältnis größer gleich 2 ist und vorzugsweise zwischen 2 und 3 liegt.

**10.** Zusammensetzung nach den Ansprüchen 8 und 9, dadurch gekennzeichnet, daß das Mo/Ni-Massenverhältnis mindestens 3/40, vorzugsweise mindestens 0,1, beträgt.

**11.** Zusammensetzung nach den Ansprüchen 8 bis 10, dadurch gekennzeichnet, daß das Ni/(Ni+Al+Mo)-Verhältnis vorteilhafterweise mindestens 0,5 beträgt.

**12.** Zusammensetzung nach den Ansprüchen 8 bis 11, dadurch gekennzeichnet, daß sie Ni/Al/Mo in den folgenden Gewichtsverhältnissen in Gew.-% enthält:

- $50 \leq Ni \leq 78$, vorzugsweise $60 \leq Ni \leq 70$;
- $20 \leq Al \leq 40$, vorzugsweise $25 \leq Al \leq 35$ und
- $5 \leq Mo \leq 20$, vorzugsweise $5 \leq Mo \leq 15$.

**13.** Hydrierungsreagenz, enthaltend eine katalytisch wirksame Zusammensetzung nach den Ansprüchen 8 bis 12 und Wasserstoff mit einem Partialdruck zwischen 1 und 100 bar, vorzugsweise zwischen 5 und 50 bar und besonders bevorzugt zwischen 15 und 25 bar (1 bar = $10^5$ Pa).